# FASCICULE DE BREVET EUROPEEN

(11) **EP 3 400 106 B1**
(45) Date de publication et mention de la délivrance du brevet: **07.08.2024**
(21) Numéro de dépôt: 17711700.9
(22) Date de dépôt: 06.01.2017
(51) Int. Cl.: B05B 7/04, B05B 7/12, B05B 11/06, A61M 15/00, A61M 15/08, A61M 11/00, B65D 83/54, B05B 11/00

(54) **ENSEMBLE DE DISTRIBUTION NASALE DE PRODUIT FLUIDE**
NASALE ABGABEANORDNUNG FÜR EIN FLÜSSIGES PRODUKT
NASAL DELIVERY ASSEMBLY FOR A FLUID PRODUCT

(30) Priorité: 07.01.2016 FR 1650121
(43) Date de publication de la demande: 14.11.2018
(73) Titulaire: Aptar France SAS, 27110 Le Neubourg (FR)
(72) Inventeur: PETIT, Ludovic, 27110 Vitot (FR)
(74) Mandataire: CAPRI
(86) Numéro de dépôt international: PCT/FR2017/050038
(87) Numéro de publication internationale: WO 2017/118825

(56) Documents cités:
- WO-A1-03/086268
- WO-A1-2004/011071
- WO-A2-02/074372
- CA-A- 985 232
- DE-A1- 102013 220 492
- FR-A1- 2 257 352
- FR-A1- 2 852 928
- GB-A- 1 493 614
- US-A- 3 921 857
- US-A- 3 923 202
- US-A- 5 437 267

## Description

La présente invention concerne un ensemble de distribution nasale de produit fluide.

Les dispositifs de distribution nasale de produit fluide sont bien connus. Ils comportent généralement un réservoir contenant une ou plusieurs doses de produit fluide, sous forme de liquide, de gel, de mousse ou de produit fluide, des moyens de distribution, tels qu'une pompe, une valve ou une chasse d'air, et une tête de distribution nasale destinée à être insérée dans une narine d'un utilisateur, ladite tête de distribution nasale comportant un orifice de distribution. Lorsque le dispositif de distribution est actionné, une dose de produit fluide est distribuée dans une narine de l'utilisateur.

Un inconvénient avec ces dispositifs de l'art antérieur concerne la distribution de la dose de produit fluide dans la narine.

De manière connue, une narine comprend notamment, en partant de l'orifice narinaire, la valve nasale, les cornets inférieur, intermédiaire et supérieur, le sinus frontal et les éthmoïdes. La valve nasale possède une géométrie particulière. Elle s'étend sur environ 1 cm de profondeur et a une section longitudinale verticale d'environ 3 à 4 cm et une largeur d'environ 1 à 3 mm. Après le passage de la valve nasale, la cavité nasale comprend une plus grande cavité (environ 7 cm de hauteur sur 2 à 3 cm de largeur). Les cornets font face à la valve nasale. Au-dessus des cornets est situé le plafond de la cavité nasale, comprenant les ethmoïdes, le bulbe olfactif et le nerf olfactif.

Les dispositifs de distribution nasale n'étant pas ou peu invasifs, le produit fluide distribué ne franchit généralement pas la valve nasale. Ainsi, de par l'anatomie de la valve nasale et de l'emplacement protectif des cornets, la trajectoire axiale ou rectiligne des particules du spray de produit fluide ne permet pas d'atteindre le plafond de la cavité nasale, et notamment les ethmoïdes.

Les documents US5437267, US3921857, GB1493614, FR2257352, WO2004011071, CA985232, FR2852928, DE102013220492 et WO02074372 décrivent des dispositifs de l'état de la technique, principalement des pompes dites biphasiques, dans lesquelles un flux d'air comprimé est mélangé au flux de liquide au niveau de l'orifice de distribution pour favoriser la pulvérisation.

La présente invention a pour but de fournir un ensemble de distribution nasale de produit fluide et/ou un procédé d'actionnement qui ne reproduisent pas les inconvénients susmentionnés.

La présente invention a aussi pour but de fournir un ensemble de distribution nasale de produit fluide et/ou un procédé d'actionnement qui améliorent le pourcentage de la dose qui atteint les éthmoïdes.

La présente invention a également pour but de fournir un ensemble de distribution nasale de produit fluide qui est simple et peu coûteux à fabriquer et à assembler.

La présente invention a donc pour objet un ensemble de distribution nasale de produit fluide selon la revendication 1. Des modes de réalisation avantageux sont décrits dans les revendications dépendantes.

Ces caractéristiques et avantages et d'autres apparaîtront plus clairement au cours de la description détaillée suivante, faite en référence aux dessins joints, donnés à titre d'exemples non limitatifs, et sur lesquels :
- la figure 1 est une vue schématique en section transversale d'un ensemble de distribution nasale de produit fluide selon un mode de réalisation avantageux, en position de repos,
- la figure 2 est une vue schématique de l'ensemble de distribution de la figure 1, après actionnement de la pompe et avant actionnement de la valve,
- la figure 3 est une vue schématique de l'ensemble de distribution des figures 1 et 2, après actionnement de la valve,
- les figures 4a et 4b sont des vues schématiques respectivement de côté et de face d'un ensemble de distribution selon un autre mode de réalisation avantageux, en position de repos,
- les figures 5a et 5b sont des vues schématiques de côté respectivement en perspective et en section d'une variante de réalisation avantageuse de l'embout nasal des ensembles de distribution des figures 1 à 4,
- la figure 6 est une vue schématique en section transversale d'un ensemble de distribution nasale de produit fluide selon un autre mode de réalisation avantageux, en position de repos,
- les figures 7a à 7c sont des vues schématiques similaires aux figures 1 à 3, illustrant encore un autre mode de réalisation avantageux,
- la figure 8 est une vue similaire à celle de la figure 7c, illustrant une variante de réalisation, et
- les figures 9a à 9c sont des vues schématiques similaires aux figures 1 à 3, illustrant encore un autre mode de réalisation avantageux.

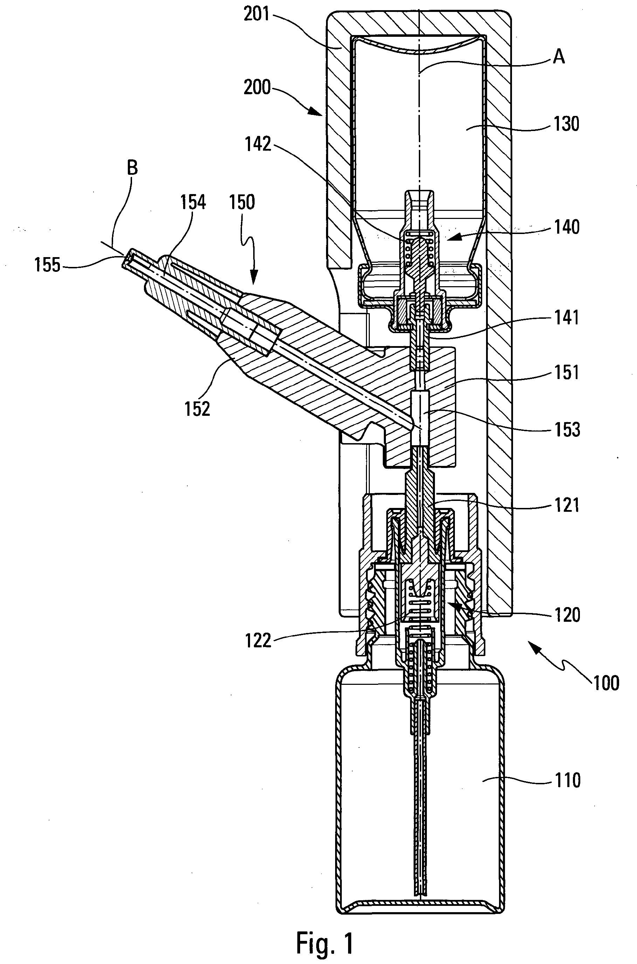

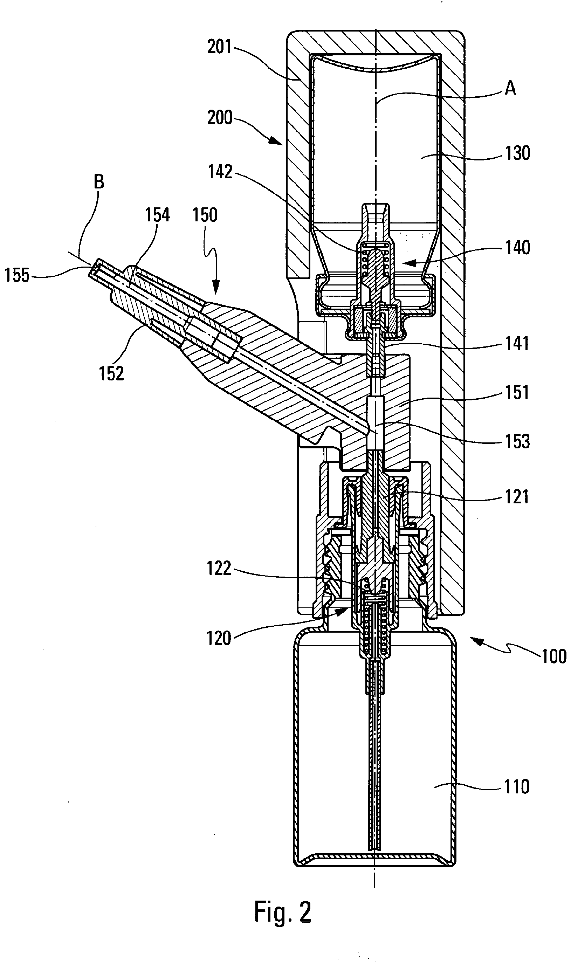

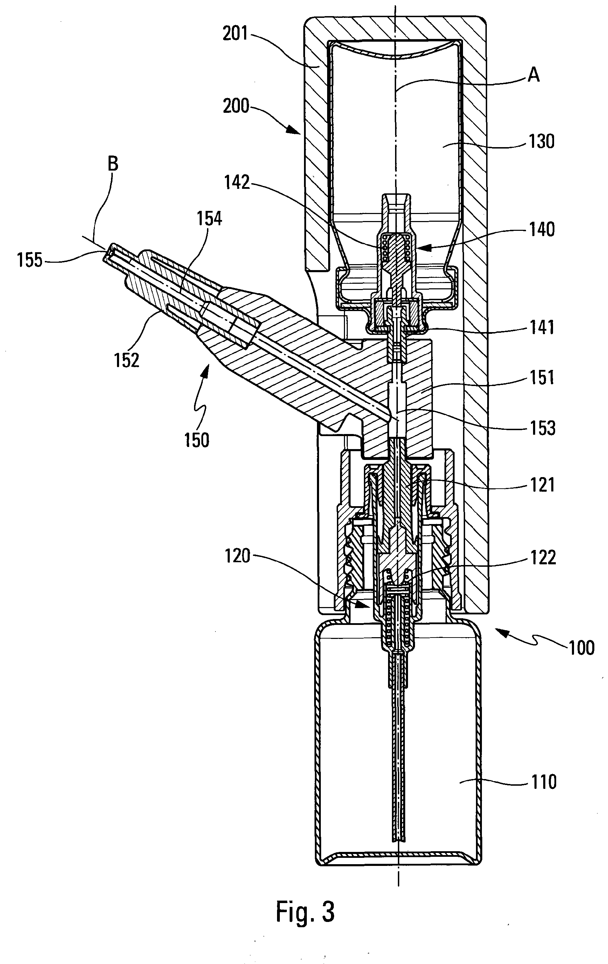

Dans la description, les termes "axial" et "radial" se réfèrent soit à l'axe longitudinal A de l'ensemble de distribution, soit à l'axe longitudinal B de l'embout nasal, ces axes longitudinaux étant représentés sur les figures 1 à 4a. Les termes "proximal" et "distal" se réfèrent à l'orifice de distribution dudit embout nasal.

Les figures 1 à 3 représentent un premier mode de réalisation avantageux.

L'ensemble de distribution nasale de produit fluide 100 comporte un premier réservoir 110 contenant une pluralité de doses d'un produit fluide, typiquement un produit fluide comportant un produit actif pharmaceutique.

Une pompe doseuse 120 est montée sur ledit premier réservoir 110 pour distribuer une dose dudit produit fluide à chaque actionnement dudit ensemble de distribution 100. De manière connue, ladite pompe doseuse 120 comporte un piston 121 qui, lors de l'actionnement dudit ensemble de distribution 100, se déplace axialement le long d'un axe longitudinal A dudit ensemble de distribution 100. La pompe doseuse 120 peut être montée sur ledit premier réservoir 110 au moyen d'une bague de fixation, par exemple vissable ou encliquetable sur le col dudit premier réservoir. La structure de la pompe doseuse 120 et sa fixation audit premier réservoir 110 peuvent toutefois être réalisées d'une quelconque manière connue, et la présente invention ne se limite pas aux exemples représentés sur les dessins.

L'ensemble de distribution 100 comporte également un système de génération d'écoulement de gaz comprimé adapté à distribuer un écoulement de gaz comprimé à chaque actionnement.

Selon un mode de réalisation préféré, représenté sur les figures 1 à 3 et 7a à 9c, ce système de génération d'écoulement de gaz comprimé comprend un second réservoir 130 contenant une pluralité de doses d'au moins un gaz propulseur pressurisé et/ou liquéfié. Avantageusement, ledit second réservoir 130 ne comporte que du gaz propulseur. En variante toutefois, ledit second réservoir 130 peut contenir, outre ledit gaz propulseur, un second produit fluide, adapté à se combiner dans la narine avec ledit produit fluide issu dudit premier réservoir 110.

Une valve doseuse 140 est montée sur ledit second réservoir 130 pour distribuer une dose de gaz propulseur à chaque actionnement dudit ensemble de distribution 100. De manière connue, ladite valve doseuse 140 comportant une soupape 141 qui, lors de l'actionnement dudit ensemble de distribution 100, se déplace axialement le long dudit axe longitudinal A. La valve doseuse 140 peut être montée sur ledit second réservoir 130 au moyen d'une capsule de fixation, par exemple sertissable sur le col dudit second réservoir. La structure de la valve doseuse 140 et sa fixation audit second réservoir 130 peuvent toutefois être réalisées d'une quelconque manière connue, et la présente invention ne se limite pas aux exemples représentés sur les dessins.

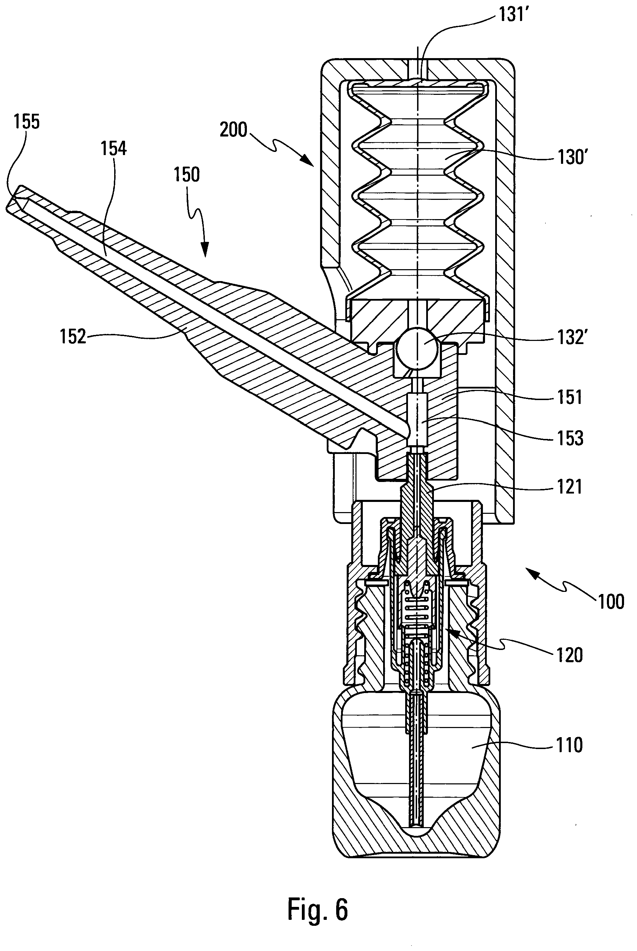

En variante à l'utilisation d'une valve doseuse montée sur un réservoir contenant du gaz propulseur, on peut envisager une chasse d'air qui à chaque actionnement comprime de l'air dans une chambre et délivre un écoulement d'air comprimé. La figure 6 illustre un exemple de chasse d'air de ce type, avec une chambre d'air 130', avantageusement formée par un soufflet. Cette chambre d'air 130' comporte de préférence un clapet d'entrée 131', ici formé par une membrane permettant une reprise d'air après chaque actionnement, et un clapet de sortie 132', ici formé par une bille. D'autres mises en oeuvre sont toutefois possibles.

L'ensemble de distribution 100 comporte en outre un embout nasal 150 comportant une partie de connexion 151 et un élément d'insertion nasale 152.

Ladite partie de connexion 151 est fixée d'un coté à la sortie de ladite pompe doseuse 120 et de l'autre coté à la sortie de ladite valve doseuse 140. Elle comportant une chambre de fluide 153 recevant à chaque actionnement dudit ensemble de distribution 100 la dose de produit fluide distribuée par ladite pompe doseuse 120 et l'écoulement de gaz comprimé distribué par ledit système de génération d'écoulement de gaz comprimé.

Ledit élément d'insertion nasale 152 comporte un corps allongé destiné à être inséré dans une narine d'un utilisateur. Ledit corps allongé s'étend longitudinalement le long d'un axe B et contient un canal de distribution 154 relié d'un côté à ladite chambre de fluide 153 et pourvu de l'autre côté d'un orifice de distribution 155 à travers lequel ladite dose de produit fluide et ledit écoulement de gaz comprimé sont distribués. Comme visible sur les figures, ledit axe B forme un angle α par rapport audit axe longitudinal A. Avantageusement, ledit angle α est supérieur à 30° et inférieur à 90°. Dans les exemples des figures 1 à 8, cet angle est de préférence environ 45°.

Selon l'invention, ladite dose de produit fluide est d'abord distribuée dans ladite chambre de fluide 153, puis ladite dose de produit fluide est ensuite expulsée sous pression par ledit écoulement de gaz comprimé hors de ladite chambre de fluide 153, à travers ledit canal de distribution 154 puis à travers ledit orifice de distribution 155.

Avantageusement, l'effort d'actionnement de ladite pompe doseuse 120 est inférieur à l'effort d'actionnement dudit système de génération d'écoulement de gaz comprimé. Ainsi, lors de l'actionnement dudit ensemble de distribution 100, ladite pompe doseuse 120 est actionnée avant ledit système de génération d'écoulement de gaz comprimé.

Avantageusement, les dimensions de la chambre de fluide 153, de la sortie de la pompe doseuse 120 et du canal de distribution 154 sont telles que lorsque l'écoulement de gaz comprimé arrive sous pression dans ladite chambre de fluide 153, la totalité (ou la quasi-totalité) de écoulement de gaz comprimé s'écoule à travers ledit canal de distribution 154 en entrainant la totalité (ou la quasi-totalité) de ladite dose de produit fluide avec lui, ce qui vide et purge non seulement ladite chambre de fluide 153 mais aussi ledit canal de distribution 154. En particulier, la dimension radiale réduite de la sortie de pompe va empêcher que du gaz comprimé et/ou du produit fluide soit réinjecté dans ladite pompe doseuse 120, l'écoulement de gaz comprimé cherchant directement le chemin de moindre résistance pour s'écouler, à savoir ledit canal de distribution 154 de l'embout nasal 150.

De manière connue, ledit piston 121 de la pompe 120 coopère avec un ressort de pompe 122 qui sollicite ledit piston 121 vers sa position de repos. De même, ladite soupape 141 de la valve 140 coopère avec un ressort de valve 142 qui sollicite ladite soupape 141 vers sa position de repos. Avantageusement, la résistance à la déformation dudit ressort de pompe 122 est inférieure à la résistance à la déformation dudit ressort de valve 142, ce qui assure un actionnement d'abord de la pompe 120 puis de la valve 140. Bien entendu, d'autres paramètres que les ressorts de la pompe 120 et de la valve 140 peuvent influencer l'effort d'actionnement.

Dans l'exemple représenté sur les figures, pour actionner ledit ensemble de pompe 100, lesdits premier et second réservoirs 110, 130 sont déplacés axialement l'un vers l'autre, sollicitant ainsi ledit piston 121 et ladite soupape 141 vers leurs positions d'actionnement respectives. C'est d'abord ledit piston 121 qui est déplacé vers sa position d'actionnement, avant que ladite soupape 141 ne soit elle aussi déplacée vers sa position d'actionnement.

En variante, pour assurer d'abord la distribution du produit fluide puis celle de l'écoulement de gaz comprimé, on peut aussi prévoir d'avoir la course d'actionnement de la pompe doseuse 120 qui est inférieure à la course d'actionnement du système de génération d'écoulement de gaz comprimé. Ainsi, en cas d'actionnements simultanés, ladite dose de produit fluide est distribuée par ladite pompe doseuse 120 avant que ledit écoulement de gaz comprimé ne soit distribué par ledit système de génération d'écoulement de gaz comprimé.

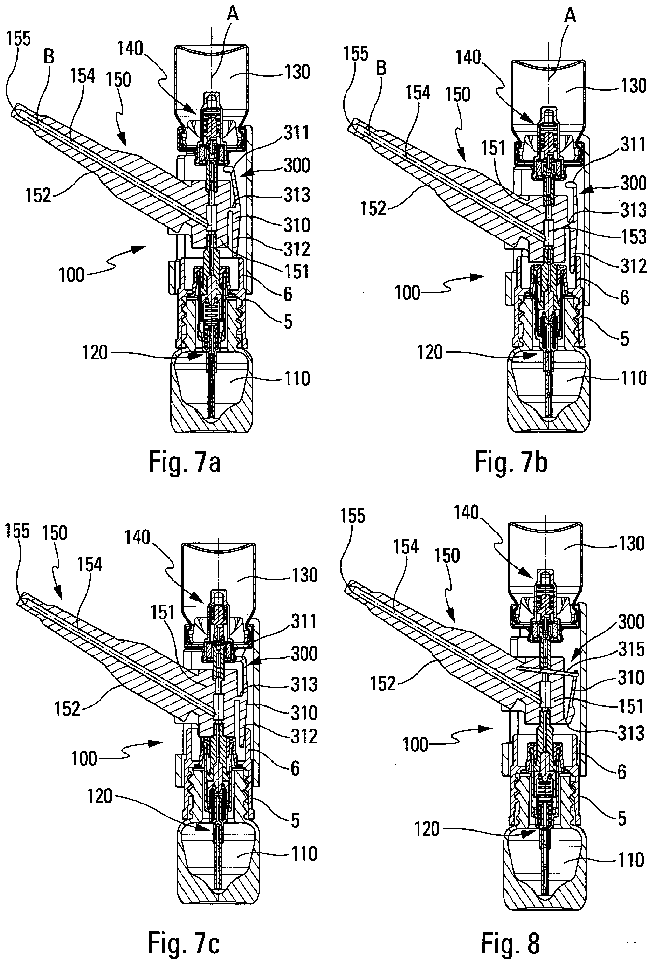

Selon une autre variante, représentée sur les figures 7a à 7c et 8, ledit système de génération d'écoulement de gaz comprimé peut comporter des moyens de verrouillage 300 qui sont libérés après distribution de ladite dose de produit fluide par ladite pompe doseuse 120. Ces moyens de verrouillage peuvent bloquer l'actionnement dudit système de génération d'écoulement de gaz comprimé et/ou bloquer la distribution dudit écoulement de gaz comprimé.

Dans l'exemple des figures 7a à 7c, les moyens de verrouillage sont formés par une tige 310 montée pivotante par une articulation 313 sur la partie de connexion 151. La tige 310 comporte une branche supérieure 311, coopérant avec le second réservoir 130 et/ou la valve 140 pour bloquer l'actionnement dudit système de génération d'écoulement de gaz comprimé. La tige 310 comporte aussi une branche inférieure 312, coopérant avec une extension axiale 6 de la bague de fixation 5 qui fixe la pompe 12 sur le premier réservoir 110. En fin de course d'actionnement de ladite pompe 120, ladite extension 6 va coopérer avec la branche inférieure 312 pour la faire pivoter autour de l'articulation 313, ce qui va automatiquement faire pivoter la branche supérieure 311 en éloignement de sa position de blocage, permettant ainsi l'actionnement dudit système de génération d'écoulement de gaz comprimé.

Dans l'exemple de la figure 8, ce n'est pas l'actionnement dudit système de génération d'écoulement de gaz comprimé qui est bloqué, mais c'est la distribution dudit écoulement de gaz comprimé qui est bloquée. Dans cet exemple, les moyens de verrouillage 300 comportent un clapet-tiroir 315 solidaire d'une tige 310 montée pivotante par une articulation 313 sur ladite partie de connexion 151. Au repos, ledit clapet-tiroir 315 obstrue la sortie de la valve 140. En fin de course d'actionnement de ladite pompe 120, ladite extension 6 de la bague de fixation 5 de la pompe 120 va coopérer avec la tige 310 pour la faire pivoter autour de l'articulation 313, ce qui va automatiquement faire pivoter la branche supérieure 311 en éloignement de sa position de blocage, ce qui provoque un coulissement dudit clapet-tiroir hors de sa position de blocage, permettant ainsi audit écoulement de gaz comprimé de s'écouler dans ladite chambre de fluide.

Pour faciliter l'actionnement, un actionneur 200 peut avantageusement être prévu. Cet actionneur 200 comporte un repose-doigts 210 pour recevoir les doigts de l'utilisateur lors de l'actionnement dudit ensemble de distribution et est fixé autour dudit second réservoir 130 et/ou de ladite valve doseuse 140. Un tel actionneur pourrait aussi être solidaire d'une chasse d'air adaptée à générer un écoulement d'air comprimé à chaque actionnement.

Lors de l'actionnement, l'utilisateur place un ou deux doigts sur ledit repose-doigts 210 et le pouce sous ledit premier réservoir 110, et sollicite ces deux éléments l'un contre l'autre. Ceci va d'abord déplacer le piston 121 et donc actionner la pompe doseuse 120, puis, lorsque la dose de produit fluide a été transférée dans ladite chambre de fluide 153, la poursuite de l'effort d'actionnement va actionner le système de génération d'écoulement de gaz comprimé, ce qui va provoquer l'expulsion d'un écoulement de gaz comprimé qui va à son tour expulser la dose de produit fluide à travers l'orifice de distribution 155.

Dans l'exemple des figures 1 à 3, ledit actionneur 200 comporte une coque creuse 201 qui contient ledit second réservoir 130 et qui entoure au moins partiellement ladite pompe doseuse 120, de sorte que ledit premier réservoir 110 fait saillie axialement hors de ladite coque creuse 201. Dans cette variante, le second réservoir 130 n'est pas visible de l'extérieur.

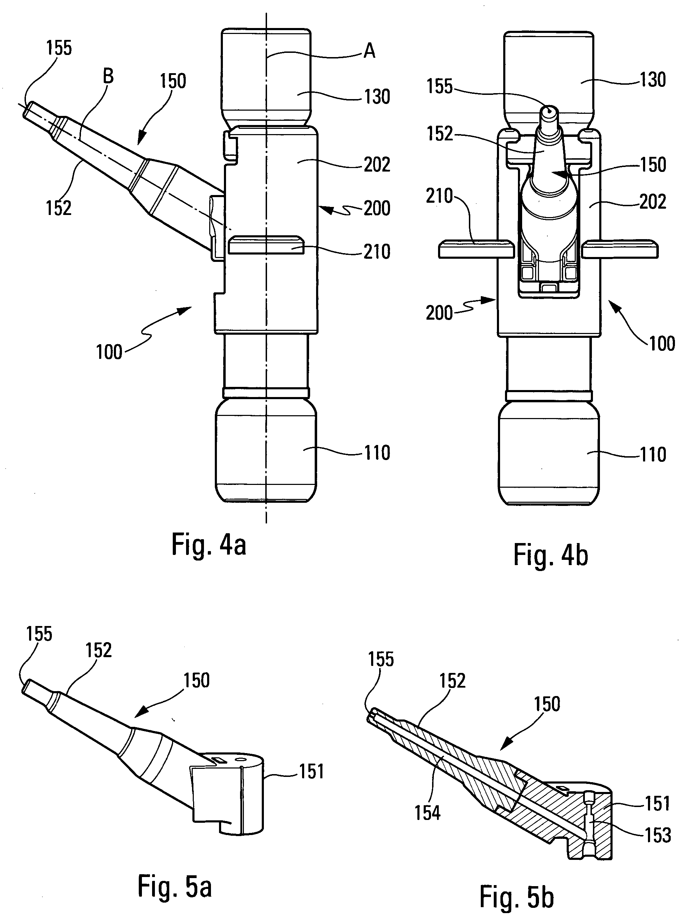

Dans l'exemple des figures 4a et 4b, ledit actionneur 200 comporte un manchon creux 202 fixé autour de ladite valve doseuse 140, par exemple au niveau de la capsule de fixation, en entourant au moins partiellement ladite pompe doseuse 120. Ici, les premier et second réservoirs 110, 130 font saillie axialement hors dudit manchon creux 202. Cette mise en oeuvre permet de réduire les dimensions extérieures dudit ensemble de distribution, et d'accéder au second réservoir 130.

Avantageusement, du fait de l'utilisation de gaz comprimé, ledit orifice de distribution 155 peut être une simple ouverture à l'extrémité axiale du canal de distribution 154, sans qu'il ne soit nécessaire de prévoir un profil de pulvérisation pour générer un spray.

Dans l'exemple des figures 1 à 3, l'embout nasal 150 est réalisé de manière rigide. Les figures 5a et 5b illustrent une variante avantageuse, dans laquelle l'élément d'insertion nasale 152 dudit embout nasal 150 est au moins partiellement réalisé en un matériau souple et/ou déformable, tel qu'un matériau thermoplastique. Avantageusement, toute la partie dudit élément d'insertion nasale 152 qui est insérée dans la narine lors de l'actionnement est réalisée avec ce matériau souple et/ou déformable. Ceci permet non seulement d'améliorer le confort de l'utilisateur, mais aussi améliore l'efficacité dudit ensemble, car un embout nasal souple peut s'insérer davantage dans la narine et peut s'auto-orienter dans la valve nasale, même si l'angle d'insertion dudit embout nasal n'est pas optimal.

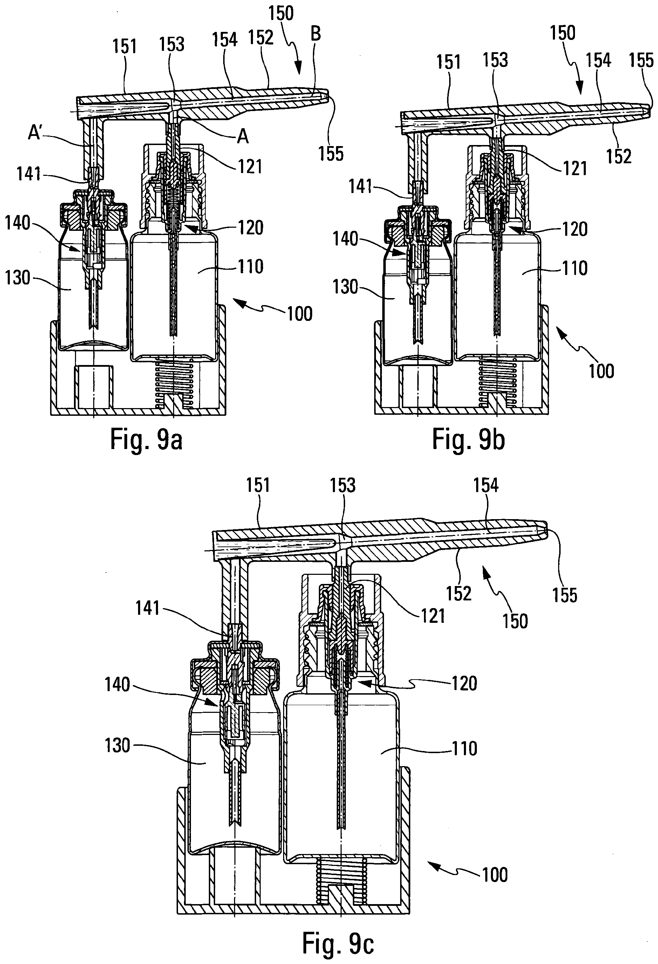

Les figures 9a à 9c illustrent encore un autre mode de réalisation avantageux, dans lequel les premier et second réservoirs 110, 130 sont disposés l'un à côté de l'autre. Dans cet exemple, la pompe 120 est donc actionnée le long d'un axe longitudinal A et la valve 140 est actionnée le long d'un axe longitudinal A', parallèle audit axe A. Comme pour les modes de réalisation précédents, l'invention prévoit de d'abord actionner la pompe 120 pour d'abord distribuer la dose de produit fluide dans la chambre de fluide 153, puis la valve 140 est actionner pour expulser ladite dose dans la narine.

## Revendications

1. Ensemble de distribution nasale de produit fluide (100) comportant:
- un premier réservoir (110) contenant une pluralité de doses d'un produit fluide, une pompe doseuse (120) étant montée sur ledit premier réservoir (110) pour distribuer une dose dudit produit fluide à chaque actionnement dudit ensemble de distribution (100), ladite pompe doseuse (120) comportant un piston (121) déplaçable axialement le long d'un axe longitudinal (A),
- un système de génération d'écoulement de gaz comprimé pour distribuer un écoulement de gaz comprimé à chaque actionnement dudit ensemble de distribution (100),
- un embout nasal (150) comportant une partie de connexion (151) et un élément d'insertion nasale (152), ladite partie de connexion (151) étant fixée d'un coté à la sortie de ladite pompe doseuse (120) et de l'autre coté à la sortie dudit système de génération d'écoulement de gaz comprimé, ladite partie de connexion (151) comportant une chambre de fluide (153) recevant à chaque actionnement dudit ensemble de distribution (100) la dose de produit fluide distribuée par ladite pompe doseuse (120) et l'écoulement de gaz comprimé distribué par ledit système de génération d'écoulement de gaz comprimé, ledit élément d'insertion nasale (152) comportant un corps allongé destiné à être inséré dans une narine d'un utilisateur, ledit corps allongé s'étendant longitudinalement le long d'un axe (B) et contenant un canal de distribution (154) relié d'un côté à ladite chambre de fluide (153) et pourvu de l'autre côté d'un orifice de distribution (155) à travers lequel ladite dose de produit fluide et ledit écoulement de gaz comprimé sont distribués, ledit axe (B) formant un angle (α) par rapport audit axe longitudinal (A),
dans lequel, ledit ensemble de distribution (100) comporte des moyens pour actionner ladite pompe doseuse (120) avant ledit système de génération d'écoulement de gaz comprimé, de telle sorte que ladite dose de produit fluide est d'abord distribuée dans ladite chambre de fluide (153), puis ladite dose de produit fluide est ensuite expulsée sous pression par ledit écoulement de gaz comprimé hors de ladite chambre de fluide (153), à travers ledit canal de distribution (154) puis à travers ledit orifice de distribution (155), **caractérisé en ce que** ladite dose de produit fluide se combine avec ledit écoulement de gaz comprimé dans ladite narine, dans lequel ledit système de génération d'écoulement de gaz comprimé comporte des moyens de verrouillage libérés après distribution de ladite dose de produit fluide par ladite pompe doseuse (120), lesdits moyens de verrouillage bloquent l'actionnement dudit système de génération d'écoulement de gaz comprimé et/ou la distribution dudit écoulement de gaz comprimé.

2. Ensemble de distribution selon la revendication 1, dans lequel l'effort d'actionnement de ladite pompe doseuse (120) est inférieur à l'effort d'actionnement dudit système de génération d'écoulement de gaz comprimé.

3. Ensemble de distribution selon l'une quelconque des revendications précédentes, dans lequel la course d'actionnement de ladite pompe doseuse (120) est inférieure à la course d'actionnement dudit système de génération d'écoulement de gaz comprimé, de sorte qu'en cas d'actionnements simultanés, ladite dose de produit fluide est distribuée par ladite pompe doseuse (120) avant que ledit écoulement de gaz comprimé ne soit distribué par ledit système de génération d'écoulement de gaz comprimé.

4. Ensemble de distribution selon l'une quelconque des revendications précédentes, dans lequel ledit système de génération d'écoulement de gaz comprimé comprend un second réservoir (130) contenant une pluralité de doses d'au moins un gaz propulseur pressurisé, une valve doseuse (140) étant montée sur ledit second réservoir (130) pour distribuer une dose de gaz propulseur à chaque actionnement dudit ensemble de distribution (100), ladite valve doseuse (140) comportant une soupape (141) déplaçable axialement le long dudit axe longitudinal (A).

5. Ensemble de distribution selon la revendication 4, dans lequel ledit piston (121) de ladite pompe doseuse (120) coopère avec un ressort de pompe (122) qui sollicite ledit piston (121) vers une position de repos, et ladite soupape (141) de ladite valve doseuse (140) coopère avec un ressort de valve (142) qui sollicite ladite soupape (141) vers ladite position de repos, la résistance à la déformation dudit ressort de pompe (122) étant inférieure à la résistance à la déformation dudit ressort de valve (142).

6. Ensemble de distribution selon la revendication 4 ou 5, dans lequel, lors de l'actionnement dudit ensemble de distribution (100), lesdits premier et second réservoirs (110, 130) sont déplacés axialement l'un vers l'autre, sollicitant ainsi ledit piston (121) et ladite soupape (141) vers des positions d'actionnement respectives, ledit ensemble de distribution comportant des moyens pour déplacer ledit piston (121) vers sa position d'actionnement avant que ladite soupape (141) ne soit déplacée vers sa position d'actionnement.

7. Ensemble de distribution selon l'une quelconque des revendications 4 à 5, dans lequel ledit second réservoir (130) contient, outre ledit gaz propulseur, un second produit fluide, adapté à se combiner dans la narine avec ledit produit fluide issu dudit premier réservoir (110).

8. Ensemble de distribution selon l'une quelconque des revendications 5 à 7, comportant un actionneur (200) fixé autour dudit second réservoir (130) et/ou dudit système de génération d'écoulement de gaz comprimé, ledit actionneur (200) comportant un repose-doigts (210) pour recevoir les doigts de l'utilisateur lors de l'actionnement dudit ensemble de distribution.

9. Ensemble de distribution selon la revendication 8, dans lequel ledit actionneur (200) comporte une coque creuse (201) qui contient ledit système de génération d'écoulement de gaz comprimé et qui entoure au moins partiellement ladite pompe doseuse (120), ledit premier réservoir (110) faisant saillie axialement hors de ladite coque creuse (201).

10. Ensemble de distribution selon la revendication 8, dans lequel ledit actionneur (200) comporte un manchon creux (202) fixé autour dudit système de génération d'écoulement de gaz comprimé et entourant au moins partiellement ladite pompe doseuse (120), ledit premier réservoir (110) et ledit système de génération d'écoulement de gaz comprimé faisant saillie axialement hors dudit manchon creux (202).

11. Ensemble de distribution selon l'une quelconque des revendications précédentes, dans lequel ledit produit fluide contenu dans ledit premier réservoir (110) comporte un produit actif pharmaceutique.

12. Ensemble de distribution selon l'une quelconque des revendications précédentes, dans lequel ledit élément d'insertion nasale (152) dudit embout nasal (150) est au moins partiellement réalisé en un matériau souple et/ou déformable, tel qu'un matériau thermoplastique.

13. Ensemble de distribution selon la revendication 12, dans lequel la partie dudit élément d'insertion nasale (152) qui est insérée dans la narine lors de l'actionnement est réalisée avec ledit matériau souple et/ou déformable.

14. Ensemble selon l'une quelconque des revendications précédentes, dans lequel ledit orifice de distribution (155) est dépourvu de profil de pulvérisation.

## Patentansprüche

1. Einheit zur nasalen Abgabe eines Fluidprodukts (100), umfassend:
- einen ersten Behälter (110), der eine Vielzahl von Dosen eines Fluidprodukts enthält, wobei am ersten Behälter (110) eine Dosierpumpe (120) montiert ist, um bei jeder Betätigung der Abgabeeinheit (100) eine Dosis des Fluidprodukts abzugeben, wobei die Dosierpumpe (120) einen Kolben (121) umfasst, der entlang einer Längsachse (A) axial bewegbar ist,
- ein System zur Erzeugung eines Druckgasstroms, um bei jeder Betätigung der Abgabeeinheit (100) einen Druckgasstrom abzugeben,
- ein Nasenstück (150), das einen Verbindungsteil (151) und ein Naseneinführelement (152) umfasst, wobei der Verbindungsteil (151) auf einer Seite am Ausgang der Dosierpumpe (120) und auf der anderen Seite am Ausgang des Druckgasstromerzeugungssystems befestigt ist, wobei der Verbindungsteil (151) eine Fluidkammer (153) aufweist, die bei jeder Betätigung der Abgabeeinheit (100) die durch die Dosierpumpe (120) abgegebene Fluidproduktdosis und den durch das Druckgasstromerzeugungssystem abgegebenen Druckgasstrom aufnimmt, wobei das Naseneinführelement (152) einen länglichen Körper aufweist, der dazu bestimmt ist, in ein Nasenloch eines Benutzers eingeführt zu werden, wobei der längliche Körper sich in Längsrichtung entlang einer Achse (B) erstreckt und einen Abgabekanal (154) enthält, der auf einer Seite mit der Fluidkammer (153) verbunden und auf der anderen Seite mit einer Abgabeöffnung (155) versehen ist, durch die die Fluidproduktdosis und der Druckgasstrom abgegeben werden, wobei die Achse (B) in Bezug auf die Längsachse (A) einen Winkel (α) bildet,
wobei die Abgabeeinheit (100) Mittel zum Betätigen der Dosierpumpe (120) umfasst, die dem Druckgasstromerzeugungssystem vorgeschaltet sind, sodass zunächst die Fluidproduktdosis in die Fluidkammer (153) abgegeben wird und anschließend die Fluidproduktdosis unter Druck durch den Druckgasstrom aus der Fluidkammer (153) durch den Abgabekanal (154) und dann durch die Abgabeöffnung (155) ausgestoßen wird,
**dadurch gekennzeichnet, dass** sich die Fluidproduktdosis im Nasenloch mit dem Druckgasstrom verbindet, wobei das Druckgasstromerzeugungssystem Verriegelungsmittel aufweist, die nach der Abgabe der Fluidproduktdosis durch die Dosierpumpe (120) freigeben werden, wobei die Verriegelungsmittel die Betätigung des Druckgasstromerzeugungssystems und/oder die Abgabe des Druckgasstroms blockieren.

2. Abgabeeinheit nach Anspruch 1, wobei die Kraft zur Betätigung der Dosierpumpe (120) kleiner als die Kraft zur Betätigung des Druckgasstromerzeugungssystems ist.

3. Abgabeeinheit nach einem der vorangehenden Ansprüche, wobei der Betätigungshub der Dosierpumpe (120) kleiner als der Betätigungshub des Druckgasstromerzeugungssystems ist, sodass bei gleichzeitiger Betätigung die Fluidproduktdosis durch die Dosierpumpe (120) abgegeben wird, bevor der Druckgasstrom durch das Druckgasstromerzeugungssystem abgegeben wird.

4. Abgabeeinheit nach einem der vorangehenden Ansprüche, wobei das Druckgasstromerzeugungssystem einen zweiten Behälter (130) umfasst, der eine Vielzahl von Dosen mindestens eines unter Druck stehenden Treibgases umfasst, wobei am zweiten Behälter (130) ein Dosierventil (140) montiert ist, um bei jeder Betätigung der Abgabeeinheit (100) eine Treibgasdosis abzugeben, wobei das Dosierventil (140) ein Ventilorgan (141) umfasst, das entlang der Längsachse (A) axial bewegbar ist.

5. Abgabeeinheit nach Anspruch 4, wobei der Kolben (121) der Dosierpumpe (120) mit einer Pumpenfeder (122) zusammenwirkt, die den Kolben (121) in eine Ruheposition vorspannt, und wobei das Ventilorgan (141) des Dosierventils (140) mit einer Ventilfeder (142) zusammenwirkt, die das Ventilorgan (141) in die Ruheposition vorspannt, wobei der Verformungswiderstand der Pumpenfeder (122) kleiner als der Verformungswiderstand der Ventilfeder (142) ist.

6. Abgabeeinheit nach Anspruch 4 oder 5, wobei bei Betätigung der Abgabeeinheit (100) der erste und der zweite Behälter (110, 130) axial aufeinander zubewegt werden, wodurch der Kolben (121) und das Ventilorgan (141) in jeweilige Betätigungspositionen vorgespannt werden, wobei die Abgabeeinheit Mittel umfasst, um den Kolben (121) in seine Betätigungsposition zu bewegen, bevor das Ventilorgan (141) in seine Betätigungsposition bewegt wird.

7. Abgabeeinheit nach einem der Ansprüche 4 bis 5, wobei der zweite Behälter (130) neben dem Treibgas ein zweites Fluidprodukt enthält, das dazu geeignet ist, sich im Nasenloch mit dem Fluidprodukt aus dem ersten Behälter (110) zu verbinden.

8. Abgabeeinheit nach einem der Ansprüche 5 bis 7, umfassend einen Aktuator (200), der um den zweiten Behälter (130) und/oder das Druckgasstromerzeugungssystem herum befestigt ist, wobei der Aktuator (200) eine Fingerauflage (210) umfasst, um die Finger des Benutzers bei Betätigung der Abgabeeinheit aufzunehmen.

9. Abgabeeinheit nach Anspruch 8, wobei der Aktuator (200) einen hohlen Mantel (201) umfasst, der das Druckgasstromerzeugungssystem enthält und die Dosierpumpe (120) zumindest teilweise umgibt, wobei der erste Behälter (110) axial aus dem hohlen Mantel (201) vorsteht.

10. Abgabeeinheit nach Anspruch 8, wobei der Aktuator (200) eine hohle Hülse (202) umfasst, die um das Druckgasstromerzeugungssystem herum angeordnet ist und die Dosierpumpe (120) zumindest teilweise umgibt, wobei der erste Behälter (110) und das Druckgasstromerzeugungssystem axial aus der hohlen Hülse (202) vorstehen.

11. Abgabeeinheit nach einem der vorangehenden Ansprüche, wobei das im ersten Behälter (110) enthaltene Fluidprodukt einen pharmazeutischen Wirkstoff enthält.

12. Abgabeeinheit nach einem der vorangehenden Ansprüche, wobei das Naseneinführelement (152) des Nasenstücks (150) zumindest teilweise aus einem weichen und/oder verformbaren Material wie einem thermoplastischen Material hergestellt ist.

13. Abgabeeinheit nach Anspruch 12, wobei der Teil des Naseneinführelements (152), der bei Betätigung in das Nasenloch eingeführt wird, aus dem weichen und/oder verformbaren Material hergestellt ist.

14. Einheit nach einem der vorangehenden Ansprüche, wobei die Abgabeöffnung (155) kein Zerstäubungsprofil aufweist.

## Claims

1. A nasal fluid dispenser assembly (100) comprising:
- a first reservoir (110) containing a plurality of doses of a fluid, a metering pump (120) being mounted on said first reservoir (110) so as to dispense a dose of said fluid each time said dispenser assembly (100) is actuated, said metering pump (120) including a piston (121) that is movable axially along a longitudinal axis (A),
- a compressed gas flow generator system for dispensing a flow of compressed gas each time said dispenser assembly (100) is actuated,
- a nasal endpiece (150) comprising a connection portion (151) and a nasal insertion element (152), said connection portion (151) being fastened at one end to the outlet of said metering pump (120), and at the other end to the outlet of said compressed gas flow generator system, said connection portion (151) including a fluid chamber (153) that, each time said dispenser assembly (100) is actuated, receives the dose of fluid dispensed by said metering pump (120), and the flow of compressed gas dispensed by said compressed gas flow generator system, said nasal insertion element (152) comprising an elongate body for inserting into a user's nostril, said elongate body extending longitudinally along an axis (B) and containing a dispenser channel (154) that is connected at one end to said fluid chamber (153), and that is provided at the other end with a dispenser orifice (155) through which said dose of fluid and said flow of compressed gas are dispensed, said axis (B) forming an angle (α) relative to said longitudinal axis (A),
wherein, said dispenser assembly (100) comprises means for actuating said metering pump (120) before said compressed gas flow generator system, such that said dose of fluid is initially dispensed into said fluid chamber (153), and then said dose of fluid is expelled under pressure by said flow of compressed gas out from said fluid chamber (153), through said dispenser channel (154), and through said dispenser orifice (155), **characterized in that** said dose of fluid combines with said compressed gas flow in said nostril, wherein said compressed gas flow generator system includes locking means that are released after said dose of fluid has been dispensed by said metering pump (120), said locking means prevent said compressed gas flow generator system from being actuated and/or said compressed gas flow from being dispensed.

2. A dispenser assembly according to claim 1, wherein the actuation force of said metering pump (120) is less than the actuation force of said compressed gas flow generator system.

3. A dispenser assembly according to any preceding claim, wherein the actuation stroke of said metering pump (120) is shorter than the actuation stroke of said compressed gas flow generator system, such that in the event of simultaneous actuations, said dose of fluid is dispensed by said metering pump (120) before said flow of compressed gas is dispensed by said compressed gas flow generator system.

4. A dispenser assembly according to any preceding claim, wherein said compressed gas flow generator system includes a second reservoir (130) containing a plurality of doses of at least one pressurized propellant gas, a metering valve (140) being mounted on said second reservoir (130) so as to dispense a dose of propellant gas each time said dispenser assembly (100) is actuated, said metering valve (140) including a valve member (141) that is movable axially along said longitudinal axis (A).

5. A dispenser assembly according to claim 4, wherein said piston (121) of said metering pump (120) co-operates with a pump spring (122) that urges said piston (121) towards a rest position, and said valve member (141) of said metering valve (140) co-operates with a valve spring (142) that urges said valve member (141) towards said rest position, said pump spring (122) resisting deformation less than said valve spring (142).

6. A dispenser assembly according to claim 4 or claim 5, wherein, during actuation of said dispenser assembly (100), said first and second reservoirs (110, 130) are moved axially towards each other, thus urging said piston (121) and said valve member (141) towards respective actuated positions, said dispenser assembly comprising means for moving said piston (121) towards its actuated position before said valve member (141) is moved towards its actuated position.

7. A dispenser assembly according to any one of claims 4 to 5, wherein said second reservoir (130), in addition to said propellant gas, contains a second fluid that is adapted to be combined in the nostril with said fluid coming from said first reservoir (110).

8. A dispenser assembly according to any one of claims 5 to 7, including an actuator (200) that is fastened around said second reservoir (130) and/or said compressed gas flow generator system, said actuator (200) including a finger rest (210) for receiving the user's fingers while actuating said dispenser assembly.

9. A dispenser assembly according to claim 8, wherein said actuator (200) comprises a hollow shell (201) that contains said compressed gas flow generator system and that surrounds said metering pump (120), at least in part, said first reservoir (110) projects axially out from said hollow shell (201).

10. A dispenser assembly according to claim 8, wherein said actuator (200) comprises a hollow sleeve (202) that is fastened around said compressed gas flow generator system and that surrounds said metering pump (120), at least in part, said first reservoir (110) and said compressed gas flow generator system project axially out from said hollow shell (202).

11. A dispenser assembly according to any preceding claim, wherein said fluid contained in said first reservoir (110) includes an active pharmaceutical.

12. A dispenser assembly according to any preceding claim, wherein said nasal insertion element (152) of said nasal endpiece (150) is made, at least in part, of a flexible and/or deformable material, such as a thermoplastic material.

13. A dispenser assembly according to claim 12, wherein the portion of said nasal insertion element (152) that is inserted into the nostril during actuation is made with said flexible and/or deformable material.

14. A dispenser according to any preceding claim, wherein said dispenser orifice (155) is devoid of spray profile
